# EUROPEAN PATENT APPLICATION

(11) **EP 4 443 198 A1**
(43) Date of publication of application: **09.10.2024**
(21) Application number: 23167192.6
(22) Date of filing: 07.04.2023
(51) Int. Cl.: G01T 1/16, G01T 1/29

(54) **X-RAY SYSTEM WITH MODULAR TWO-LAYER DETECTOR**

(71) Applicant: Koninklijke Philips N.V., 5656 AG Eindhoven (NL)
(72) Inventor: JOHNSON, Mark Thomas, Eindhoven (NL); VOGTMEIER, Gereon, Eindhoven (NL); KOEHLER, Thomas, 5656AG Eindhoven (NL); CHAUDHURY, Sudipta, Eindhoven (NL); CHAKRABARTI, Biswaroop, Eindhoven (NL)
(74) Representative: Philips Intellectual Property & Standards

(57) **Abstract**

The present invention relates to a detection system for an X-ray imaging system. The detection system comprises a first detector a second detector, each detector comprising a detector area and being configured for detecting X-ray radiation emitted by an X-ray source of the X-ray imaging system impinging onto the respective detection area. The detection system is configured to adjust a position of at least one of the first detector and the second detector with respect to the other of the first detector and the second detector. The first detection area of the first detector and the second detection area of the second detector can be positioned at least partially one behind the other with respect to a direction of X-ray radiation emitted from the X-ray source of the X-ray imaging system.

## Description

### FIELD OF THE INVENTION

The present invention relates to a detection system for an X-ray imaging system, an X-ray imaging system comprising the detection system, and a method of imaging a subject with the X-ray imaging system.

### BACKGROUND OF THE INVENTION

Imaging in the diagnostic or interventional X-ray imaging area (DXR / IXR) is usually mainly limited to the detection of the linear attenuation coefficient at the mean energy of the X-ray beam. However, in the area of computed tomography, there is the trend to move to spectral imaging using various different methods, like dual-source imaging, dual layer detectors, fast kVp-switching, or even photon counting with energy discrimination. Spectral imaging allows to differentiate the two contributions to attenuation, namely photo-electric absorption and Compton scattering. This allows in the end for instance to separate soft tissue from iodine in the image, leading in some tasks to an improved or simplified diagnosis. Dual layer detectors can be used for spectral imaging, with two fixed layers that are used for energy separation detecting low energy photons on the top layer and high energy photons on the bottom layer. However, as spectral imaging is currently only a niche application in DXR and IXR, large dual layer detectors are prohibitively expensive.

The inventors of the present invention have thus found that it would be advantageous to have an improved detection system for an X-ray imaging system that at least partially solves these problems and allows spectral X-ray imaging while reducing costs.

### SUMMARY OF THE INVENTION

It is an object of the present invention to provide an improved modular detection system for an X-ray imaging system that allows flexible adaption of the detection area and spectral X-ray imaging on at least a part of the active detection area.

The object of the present invention is solved by the subject matter of the independent claims, wherein further embodiments are incorporated in the dependent claims.

The described embodiments similarly pertain to the detection system for the X-ray imaging system, the X-ray imaging system comprising the detection system, and the method of imaging a subject with the X-ray imaging system. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a detection system for an X-ray imaging system. The detection system comprises a first detector configured to detect X-ray radiation of the X-ray imaging system impinging onto a first detection area of the first detector, and a second detector configured to detect X-ray radiation of the X-ray imaging system impinging onto a second detection area of the second detector. The detection system is configured to adjust a position of at least one of the first detector and the second detector with respect to the other of the first detector and the second detector, and to position the first detection area of the first detector and the second detection area of the second detector at least partially one behind the other with respect to a direction of the X-ray radiation impinging onto the first detection area and the second detection area.

Thus, the invention proposes a modular DXR or IXR detection system comprising a first detector adapted to be situated adjacent to a subject being scanned, and a second detector situated at least partially behind the first detector and at least partially shielded from the subject being scanned by the first detector. Both detectors detect X-ray radiation produced by a generator or source of the X-ray imaging system that passes through the subject and impinges onto the detection system. The second detector can be repositioned relative to the first detector. Thus, a detector configuration with two at least partially overlapping detector layers is provided, where both detector layers are detecting signals that are used to form the final X-ray image, and where the two detector layers can be repositioned with respect to each other to adapt the detection area and/or properties of the detector.

For example, for an energy separation mode, the second detector that may be smaller than the first detector can be placed at a region of interest to provide spectra imaging in that region, while the first detector acquires information for a full body image. Preferably, the first detector and the second detector can differ in pixel size, spatial resolution, and/or energy sensitivity, etc. Thus, advantageously the detection areas of two detectors that have been slid behind each other, where one detector is shielded by the other from the subject being scanned, are used for image reconstruction.

In an embodiment of the invention, the first detection area and the second detection area are arranged essentially parallel or concentrically to each other. This ensures that radiation emitted from the X-ray source penetrates both the first detection area and the second detection area and is thus detected by both detectors. The detection areas may have a plane or a curved surface. In the case of curved surfaces, the detectors may be arranged concentrically.

In an embodiment of the invention, the second detector is configured to be translated relative to the first detector in a direction parallel to a surface of the first detection area of the first detector and/or in a direction perpendicular to the surface of the first detection area of the first detector. Optionally, at least one of the detectors may be a curved main detector, and the smaller detector might follow the curved geometry of the main detector when translated. In this case, it may be necessary to tilt the smaller detector to adjust the alignment with the main detector.

In an embodiment of the invention, a size of the second detection area of the second detector differs from a size of the first detection area of the first detector.

In an embodiment of the invention, a spatial resolution of the second detector differs from a spatial resolution of the first detector.

In an embodiment of the invention, a transparency of the first detector with respect to of the X-ray radiation impinging onto the first detection area and the second detection area is higher than a transparency of the second detector with respect to the X-ray radiation impinging onto the first detection area and the second detection area.

In an embodiment of the invention, an energy sensitivity of the second detector differs from an energy sensitivity of the first detector. Thus, the first detector and the second detector may preferably detect X-rays with different wavelength.

In an embodiment of the invention, the detection system further comprises a processing unit configured to reconstruct an X-ray image based on X-ray radiation that is detected by the first detector and the second detector.

In an embodiment of the invention, the reconstructed X-ray image comprises a first region and a second region, wherein the first region is reconstructed based on X-ray radiation that is detected by both the first detector and the second detector, and the second region is reconstructed based on X-ray radiation that is detected by one of the first detector and the second detector, and at least one image characteristic of the X-ray image is better in the first region than in the second region. Thus, the first region can enable spectral imaging, a higher spatial resolution, higher image quality due to less scattering, etc. Optionally, the exact position of the first detector and/or the second detector can be measured and the position information can be used for example for the image reconstruction as the position might vary over the time of the scan.

In an embodiment of the invention, at least one of the first detector and the second detector comprises an anti-scatter grid.

According to another aspect of the invention, there is provided an X-ray imaging system comprising the detection system according to any of the preceding embodiments and an X-ray source.

In an embodiment of the invention, the X-ray imaging system comprises a beam collimator, and the beam collimator is configured to focus the X-ray radiation emitted by the X-ray source to an area covered by the first detection area and the second detection area. The adaptation of the beam collimation with a tube collimator may be necessary to adapt to the beam to the detector size and the imaged body region.

In an embodiment of the invention, the X-ray imaging system further comprises an irradiation indicator, the irradiation indicator being configured to visualize an anatomical region of a subject to be imaged, the anatomical region being located between the X-ray source and at least one of the first detection area and the second detection area. For example, a laser beam can be used to indicate the borders of the detection areas on the subject.

In an embodiment of the invention, the irradiation indicator is configured to separately visualize an anatomical region being located between the X-ray source and the first detection area and an anatomical region being located between the X-ray source and the second detection area.

According to another aspect of the invention, there is provided a method of imaging a subject with the X-ray imaging system according to any of the preceding embodiments. The method comprises the steps of providing the X-ray imaging system, positioning the subject between the X-ray source and the detection system, adjusting the position of at least one of the first detector and the second detector of the detection system, and acquiring an image of at least an anatomical region of the subject. Preferably, a region of interest of the subject is imaged with increased quality and/or different imaging properties.

Thus, the benefits provided by any of the above aspects equally apply to all of the other aspects and vice versa.

In summary, the invention relates to a detection system for an X-ray imaging system. The detection system comprises a first detector a second detector, each detector comprising a detector area and being configured for detecting X-ray radiation emitted by an X-ray source of the X-ray imaging system impinging onto the respective detection area. The detection system is configured to adjust a position of at least one of the first detector and the second detector with respect to the other of the first detector and the second detector. The first detection area of the first detector and the second detection area of the second detector can be positioned at least partially one behind the other with respect to a direction of X-ray radiation emitted from the X-ray source of the X-ray imaging system.

One of the advantages of embodiments of the present invention of the detection system with flexible geometry configuration is that a region of interest of the patient can be imaged with spectral X-ray imaging. Another advantage is that a field of view of the X-ray system, in particular the overall detection area, can be extended by using two partially overlapping detectors. Another advantage is that an adaption of the spatial resolution of the detection system can be applied, leading to a zoom function of the detection system. Another advantage is that if the second detector is applied with a focused anti-scatter grid, scattered radiation produced by the first detector reaching the second detector can be reduced.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Figs. 1A to 1C show schematic setups of a detection system for an X-ray imaging system according to an embodiment of the invention.
Fig. 2 shows a schematic setup of an X-ray imaging system comprising the detection system according to an embodiment of the invention.
Fig. 3 shows a block diagram of a method of imaging a subject with the X-ray imaging system according to an embodiment of the invention.

### DETAILED DESCRIPTION OF EMBODIMENTS

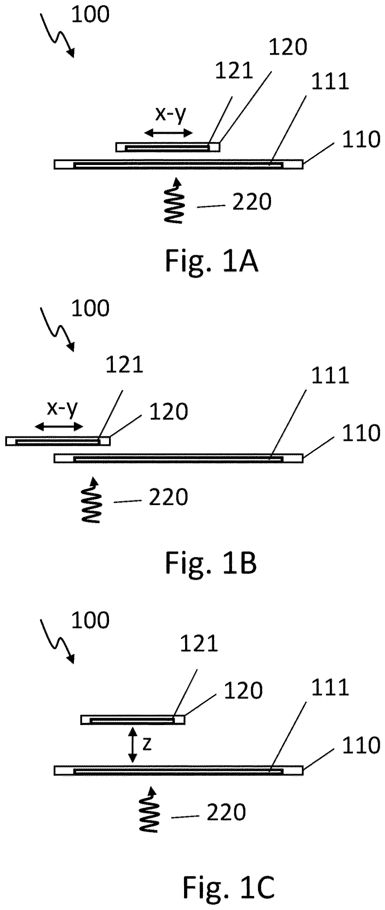

Figs. 1A to 1C show schematic setups of a detection system 100 for an X-ray imaging system 200 according to an embodiment of the invention. Different possible geometrical relationships between the two detectors for a diagnostic X-ray imaging system are indicated.

In a first exemplary embodiment shown in Fig. 1A, a complete overlap between the first detection area 111 of the first detector 110 and the second detection area 121 of the second detector 120 is shown. The detection areas of both detectors are arranged parallel to each other, and are configured for detecting X-ray radiation 220 emitted from a radiation source 210 of the X-ray imaging system 200. The X-ray radiation 220 penetrates the first detection area 111 and the second detection area 121 and is detected in both detectors, which is possible due to at least a partial overlap of the respective detection areas 111, 121. The signals of both detectors are used for reconstruction of an X-ray image. In this embodiment, at least the second detector 120 is configured such that a position of the second detector 120 with respect to the first detector 110 can be adjusted. This repositioning is indicated by the double-arrow next to the second detector, which indicates a transversal translation in a x-y-direction parallel to the first detection area 111. This allows to select a region of interest of a subject 140 to be imaged, where an area of overlap is realized where both detectors detect the X-rays penetrating the area of overlap. Imaging outside the overlap area is performed with just a single detector, i.e. the first detector 110 in this embodiment.

In a second exemplary embodiment shown in Fig. 1B, a configuration is shown with only a partial overlap between the first detection area 111 and the second detection area 121. Translating the second detector 120 to the border of the first detector 110 may lead to this partial overlap. However, X-ray radiation 220 still penetrates both the first detection area 111 and the second detection area 121 at least partially. In this configuration, the complete area of the field where the X-rays may be detected in the X-ray imaging system may be extended. Additionally, a region of overlap can be realized, where both detectors detect the X-ray radiation 220.

In a third exemplary embodiment shown in Fig. 1C, a configuration of the detection system 100 is shown, in which additionally the distance between the first detector 110 and the second detector 120 in the z-direction can be adjusted. Thus, movement in the plane of the detector and perpendicular to the plane is possible. This may have the advantage that the second detector 120 picks up less scattered radiation generated in the first detector 110. Specifically, after a certain distance the scattered radiation detected by the second detector 120 will be less, while direct unscattered high energy X-ray radiation will still reach the second detector 120.

It is noted, that whilst the figures depict a situation where the first detector 110 and in particular the first detection area 111 is larger than the second detection area 121 of the second detector 120. However, in other not illustrated embodiments the second detector 120 may exceed the first detector in size.

Whilst there may be advantages of using two detectors with identical properties, the proposed concept can be further enhanced by the implementation of two detectors with different properties. Hence, the following combinations can be considered.
- Transparency: When the two detectors overlap each other, the overlapping part can be used for a double detection of X-ray radiation 220 penetrating the overlapping part. Thus, it is advantageous if the first detector 110 has a higher transparency for the applied radiation than the second detector 120.
- Resolution: It may be advantageous if the first detector 110 has a different spatial resolution than the spatial resolution of the second detector 120. For example, the first detector 110 may be a smaller, higher resolution detector, whilst the second detector 120 is a larger, lower resolution detector. In such a configuration a higher resolution image of a region of interest can be realized in a larger field of view with lower resolution.
- Spectral: It may be advantageous if the first detector 110 has a different spectral feature to that of the second detector 120. For example, the first detector 110 may be sensitive to X-rays at a given first wavelength, while the second detector 120 is a sensitive to X-rays of a given second wavelength. In such a configuration, spectral imaging can be realized. Preferably, the first detector 110 is more sensitive to low-energy X-rays with a long wavelength, while the second detector 120 is more sensitive to high-energy X-rays with a shorter wavelength.

In general, any combination of the above-mentioned different properties can be considered. For example, two detectors with different functionalities like spectral sensitivity and spatial resolution and a double detection function at the overlapping area can be employed. This might lead to a higher resolution of the resulting X-ray image at one wavelength and a lower resolution at a second wavelength.

Preferably, the first detection area 111 and the second detection area 121 are arranged essentially parallel or concentrically to each other. This ensures that radiation emitted from the X-ray source 210 penetrates both the first detection area 111 and the second detection area 121 and is thus detected by both detectors. The detection areas may have a plane or a curved surface. In the case of plane surfaces, the detectors may be arranged parallel or at least essentially parallel to each other. In the case of curved surfaces, the detectors may be arranged concentrically.

Preferably, the second detector 120 is movable such that it can be translated relative to the first detector 110 in a direction parallel and/or perpendicular to the surface of the first detection 111 area of the first detector 120. Optionally, at least one of the detectors may be a curved main detector, and the smaller detector might follow the curved geometry of the main detector when translated. In this case, it may be necessary to tilt the smaller detector to adjust the alignment with the main detector.

In embodiments of the invention, the detection system 100 comprises a processing unit 130 that communicates with the first detector 110 and the second detector 120, and generates an X-ray image based on the X-ray radiation 220 that is detected by the first detector 110 and the second detector 120.

Preferably, the generated or reconstructed X-ray image comprises a first region and a second region. The first region can be reconstructed based on X-ray radiation 220 that is detected by both the first detector and the second detector, whereas the second region is reconstructed based on X-ray radiation 220 that is detected by only one of the first detector and the second detector. Thus, the first region is the area where the first detection area 111 and the second detection area 121 are overlapping each other, such that at least one image characteristic of the X-ray image can be different, preferably better in the first region than in the second region. Thus, the first region can enable at least one of spectral imaging, a higher spatial resolution, higher image quality due to less scattering, etc. Optionally, the exact position of the first detector 110 and/or the second detector 120 can be measured and the position information can be used for example for the image reconstruction as the position might vary over the time of the X-ray scan.

Further, at least one of the detectors, preferably the second detector 120 can comprise an anti-scatter grid for suppressing radiation that is scattered in the first detector 110.

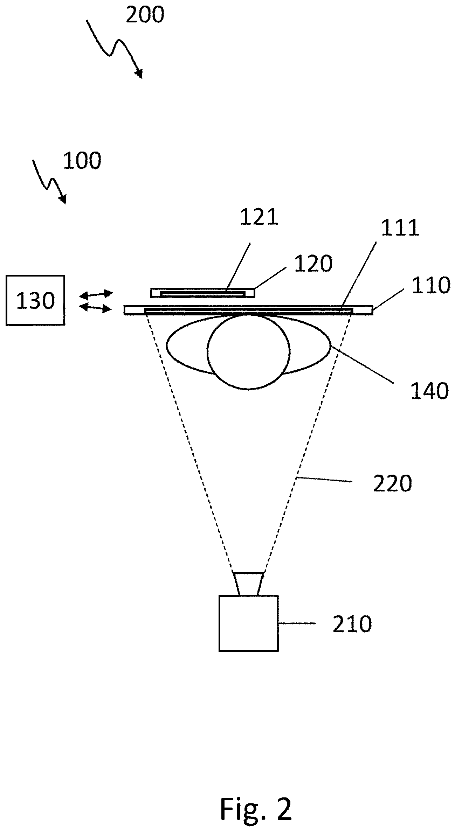

Fig. 2 shows a schematic setup of an X-ray imaging system 200 comprising the detection system 100 and an X-ray source 210 according to an embodiment of the invention. A patient is position as subject 140 to be imaged between the detection system 100 and the X-ray source 210 of the X-ray imaging system 200. X-ray radiation 220 emitted from the X-ray source 210 penetrates the subject 140 and both the first detection rea 111 of the first detector 110 and the second detection area 121 of the second detector 120. A processing unit 130 is used for readout of the information received from the two detectors and generates an X-ray image of the subject 140 using data from both detectors.

Optionally, the X-ray imaging system 200 can comprise a beam collimator for focusing the X-ray radiation 220 emitted by the X-ray source 210 to an area covered by the first detection area 111 and the second detection area 121. The adaptation of the beam collimation with a tube collimator may be necessary to adapt to the beam to the detector size and the imaged body region. Further, an irradiation indicator can be used to visualize an anatomical region of the subject 140 to be imaged. For example, a laser beam can be used to indicate the borders of the detection areas or the shape of the X-ray beam on the subject 140. Preferably, the irradiation indicator can separately visualize an anatomical region being located between the X-ray source 210 and the first detection area 111 and an anatomical region being located between the X-ray source 210 and the second detection area 121.

Thus, a collimator can be used to limit the X-ray beam geometrically such that only the relevant anatomical area of the patient is irradiated. This is usually done by means of optics that casts visible light in the beam, allowing an operator to adjust the collimator properly. With the detection system having two areas with different features, it is desired to indicate to the operator separately the areas where spectral and conventional imaging is possible. One possible option therefore may be to use a beam indicator that visualizes the positions of the collimator blades of the beam collimator by four laser lines. In the present case, the positions of the detector edges of both detectors can be visualized by four additional laser lines, preferably using a different color than that the first four laser lines.

Optionally, the second detector 120 can be placed at an angle of approximately 59° with respect to the central axis of the beam on the same arc to capture maximum scatter information as the scattering peaks are at the aforesaid angles. A suitably reconstructed volume from the second detector 120 can be used as a scattering mask, which in turn can be applied on the primary volume acquired by the first detector 110 in order to remove scattering artifacts. The above-mentioned arrangement of the second detector 120 with an angle of 59° w.r.t. the central beam can also be used to supplement the primary volume with the scatter information.

Further, in case the first detector 110 and the second detector 120 differ in pixel dimension, or even if they have the same spatial resolution but are deliberately imprecisely aligned, the image corresponding to the overlapping region can be reconstructed with higher resolution than either the first detector 110 or the second detector 120 would have allowed using super-resolution techniques for image reconstruction.

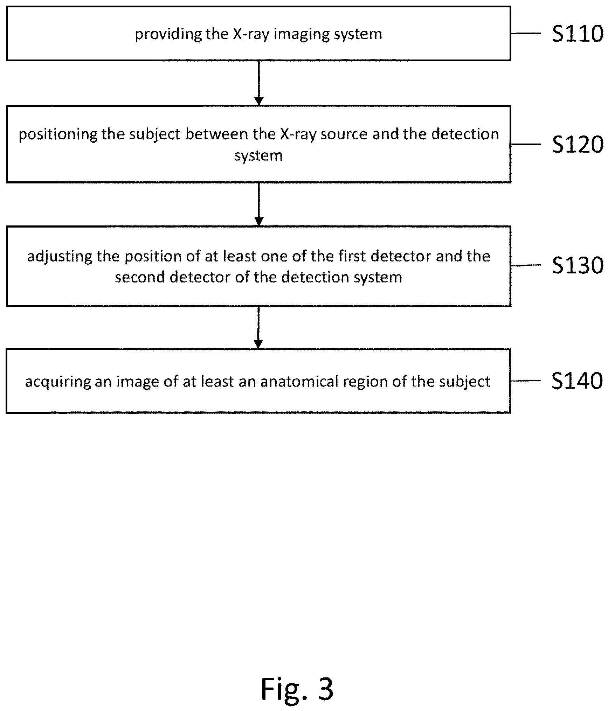

Fig. 3 shows a block diagram of a method of imaging a subject 140 with the X-ray imaging system 200 according to an embodiment of the invention. The method comprises the step S110 of providing the X-ray imaging system 200, the step S120 of positioning the subject 140 between the X-ray source 210 and the detection system 100, the step S130 of adjusting the position of at least one of the first detector 110 and the second detector 120 of the detection system 100, and the step S140 of acquiring an image of at least an anatomical region of the subject 140. Thus, preferably a region of interest of the subject 140 is imaged with increased quality and/or different imaging properties like spectral imaging.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: detection system
- 110: first detector
- 111: first detection area
- 120: second detector
- 121: second detection area
- 130: processing unit
- 140: subject
- 200: X-ray imaging system
- 210: X-ray source
- 220: X-ray radiation

## Claims

1. A detection system (100) for an X-ray imaging system (200), the detection system comprising
a first detector (110) configured to detect X-ray radiation (220) of the X-ray imaging system (200) impinging onto a first detection area (111) of the first detector (110);
a second detector (120) configured to detect X-ray radiation (220) of the X-ray imaging system (200) impinging onto a second detection area (121) of the second detector (120);
wherein the detection system (100) is configured to adjust a position of at least one of the first detector (110) and the second detector (120) with respect to the other of the first detector (110) and the second detector (120); and
wherein the detection system (100) is configured to position the first detection area (111) of the first detector (110) and the second detection area (121) of the second detector (120) at least partially one behind the other with respect to a direction of the X-ray radiation (220) impinging onto the first detection area (111) and the second detection area (121).

2. The detection system (100) according to claim 1, wherein the first detection area (111) and the second detection area (121) are arranged essentially parallel or concentrically to each other.

3. The detection system (100) according to any of claims 1 or 2, wherein the second detector (120) is configured to be translated relative to the first detector (110) in a direction parallel to a surface of the first detection area (111) of the first detector (110) and/or in a direction perpendicular to the surface of the first detection area (111) of the first detector (110).

4. The detection system (100) according to any of the preceding claims, wherein a size of the second detection area (121) of the second detector (120) differs from a size of the first detection area (111) of the first detector (110).

5. The detection system (100) according to any of the preceding claims, wherein a spatial resolution of the second detector (120) differs from a spatial resolution of the first detector (110).

6. The detection system (100) according to any of the preceding claims, wherein a transparency of the first detector (110) with respect to of the X-ray radiation (220) impinging onto the first detection area (111) and the second detection area (121) is higher than a transparency of the second detector (120) with respect to the X-ray radiation (220) impinging onto the first detection area (111) and the second detection area (121).

7. The detection system (100) according to any of the preceding claims, wherein an energy sensitivity of the first detector (110) differs from an energy sensitivity of the second detector (120).

8. The detection system (100) according to any of the preceding claims, further comprising a processing unit (130) configured to reconstruct an X-ray image based on X-ray radiation (220) that is detected by the first detector (110) and the second detector (120).

9. The detection system (100) according to claim 8, wherein the reconstructed X-ray image comprises a first region and a second region, wherein the first region is reconstructed based on X-ray radiation (220) that is detected by both the first detector (110) and the second detector (120), and wherein the second region is reconstructed based on X-ray radiation (220) that is detected by one of the first detector (110) and the second detector (120), and wherein at least one image characteristic of the X-ray image is better in the first region than in the second region.

10. The detection system (100) according to any of the preceding claims, wherein at least one of the first detector (120) and the second detector (120) comprises an anti-scatter grid.

11. An X-ray imaging system (200) comprising the detection system (100) according to any of claims 1 to 10 and an X-ray source (210).

12. The X-ray imaging system (200) according to claim 11, further comprising a beam collimator, wherein the beam collimator is configured to focus the X-ray radiation (220) emitted by the X-ray source (210) to an area covered by the first detection area (111) and the second detection area (121).

13. The X-ray imaging system (200) according to any of claims 11 or 12, further comprising an irradiation indicator, wherein the irradiation indicator is configured to visualize an anatomical region of a subject (140) to be imaged, the anatomical region being located between the X-ray source (210) and at least one of the first detection area (111) and the second detection area (121).

14. The X-ray imaging system (200) according to claim 13, wherein the irradiation indicator is configured to separately visualize an anatomical region of the subject (140) being located between the X-ray source (210) and the first detection area (111) and an anatomical region of the subject (140) being located between the X-ray source (210) and the second detection area (121).

15. A method of imaging a subject (140) with the X-ray imaging system (200) according to any of claims 11 to 14, the method comprising the steps of:
providing the X-ray imaging system (200);
positioning the subject (140) between the X-ray source (210) and the detection system (100);
adjusting the position of at least one of the first detector (110) and the second detector (120) of the detection system (100); and
acquiring an image of at least an anatomical region of the subject (140).
